# EUROPEAN PATENT APPLICATION

(11) **EP 4 600 616 A1**
(43) Date of publication of application: **13.08.2025**
(21) Application number: 24382127.9
(22) Date of filing: 09.02.2024
(51) Int. Cl.: G01F 11/02, G01F 15/00, A61F 9/00

(54) **A DISPENSER FOR DOSES OF LIQUID OR PASTY SUBSTANCES**

(71) Applicant: Brill Engines, S.L., 08022 Barcelona (ES)
(72) Inventor: BUISAN FERRER, Josep, 08006 BARCELONA (ES); NIETO CAVIA, Laura, 08027 BARCELONA (ES); BOSCH RIBES, Enric, 08859 BEGUES (ES)
(74) Representative: ABG Intellectual Property Law, S.L.

(57) **Abstract**

The present invention relates to a dispenser (100) for doses of liquid or pasty substances (1) that has a receptacle (2) for storing the substance to be dispensed and a piston (4) telescopically fitted into the receptacle (2), the piston prevented from rotating by an anti-rotation mechanism (5) and threadedly coupled in a rotating sleeve (3) around an axis of rotation (x). The dispenser has an actuator (6), manually operable for the movement thereof, and transmission means (7) that connect the actuator (6) with the rotating sleeve (3). The actuator (6) is able to move forward and backward in a first (A) and a second (B) direction of movement, respectively, between two stroke stops; and the transmission means (7) comprise a pushing part (71) in the actuator (6) and a pushed part (72) in the rotating sleeve (3), configured so that the pushed part (72) receives a push with a component that causes a rotation of the rotating sleeve (3) in the direction that moves the piston (4) against the substance stored in the receptacle (2) when the actuator (6) is moved between its stroke stops, both forward and backward.

## Description

### Technical field of the invention

The invention relates to a dispenser for doses of liquid or pasty substances, such as ophthalmological or dermatological substances, respectively, stored in a receptacle the volume of which decreases as doses of said substance are released.

### Background of the invention

Currently, devices are known for dispensing doses of liquid or pasty substances that use different techniques for said dispensing.

A known technique is pumping without air intake (airless pump), which is essentially based on storing the substance such that it occupies a receptacle having a variable volume under pressure conditions such that when the receptacle is connected to the outside, and a dose of the substance that fills the same is evacuated, a piston thrust by atmospheric pressure moves towards the remaining substance, reducing the volume of the receptacle, to compensate for the volume of the evacuated substance. This technique requires that the piston can only move in the direction that reduces the volume of the receptacle.

Other known techniques are based on producing the movement of a piston encapsulated in the receptacle that stores the substance and against the aforementioned substance, raising its pressure until it reaches a threshold value that causes its evacuation from the receptacle. For the implementation of these techniques, it is also of interest that the piston can only move in the direction that goes against the substance to be evacuated.

It is for this reason that the piston is sometimes referred to as a non-return element or piston.

There are several non-return solutions. A common non-return solution is based on equipping the piston with a series of tabs strategically placed on the periphery of the piston and configured to be elastically deformed allowing the piston to slide with adjustment through an enveloping sleeve in the direction that reduces the volume of the receptacle but that interlock and do not allow the same piston to slide in the opposite direction.

This solution is viable when the volume, and therefore the dimensions of the dispenser, are large, but it is a problematic solution when the dimensions of the dispenser are small. Dispensers having small dimensions may be required when they are intended to be held and operated with a single hand for the evacuation of a dose of the stored substance directly onto a specific target body part, for example, over an eye, instead of evacuating a non-metered amount of substance on the hands and then use the hands to distribute the substance. As an example, an ophthalmological substance may be contaminated if it is not applied directly to the eye and low volume doses are usually required.

The aforementioned problems are problems of scale and manufacturing tolerances. For example, the tabs that perform the non-return function are very small in size and it becomes difficult and/or too expensive to manufacture the associated parts and ensure their correct functioning once the dispensing device is assembled.

Another non-return solution is based on the use of pistons threaded into an outer sleeve so that, by way of a spindle, the piston advances in the direction towards the receptacle when a rotation is transmitted to the outer sleeve. To do this, it is required that the piston be prevented from rotating so that the movement imparted to the piston is a translational movement. It is for this reason that the piston is sometimes referred to as a telescopic element or piston.

Patent document US 7309184 discloses a liquid substance dispensing device in which a telescopic piston is advanced through the inside of a substance storage receptacle. The piston is threadedly coupled to a threaded stem engaged with an outer actuator, so that by rotating the outer actuator, the stem is rotated and the advancement of the piston through the inside of the receptacle and against the substance is achieved, producing its expulsion.

This type of dispenser has the drawback that it is not specially prepared to dispense doses of the substance, there is no control of the volume of the substance evacuated: the more the outer actuator is rotated, the greater the movement of the piston and the amount of substance evacuated.

Patent document EP 4106577 teaches a dispenser for creams or gels that is conceptually similar to that disclosed in US 7309184 but that incorporates an interlocking system, by way of a ratchet, which operates between components with a relative rotation movement between each other, one of which is rotated by a user. The locking system is formed by an elastically deformable tab with a harpoon head in one of the components that coincides with a housing provided in the other component when they adopt a certain relative angular position, the harpoon head then being housed in the housing by elastic restoring force of the tab. This housing causes a braking that warns the user that a specific relative movement has occurred between the components that translates into a specific movement of the piston and in turn into a dose of the substance. The harpoon head is configured, together with the associated housing, to be dislodged from the housing when a sufficient torque is applied in the appropriate direction to one of the relative movement components and a dose of substance can be evacuated again.

This type of dispenser has the drawback that it is not suitable for being held and manoeuvred with a single hand. In the case of ophthalmological substances, it is not ergonomic to have to apply a rotational movement to a small outer rotating actuator, when at the same time accuracy is required so that the dose of released substance falls directly on an eye. This type of dispenser has other drawbacks which, since they are common to the devices described below, will be explained later.

Patent document CN204863997 teaches a dispensing device that is conceptually similar to that which is disclosed in US 7309184, which has a graduated window to observe the substance to be dispensed and the outer rotating actuator of which is also graduated to be able to know the amount of rotation that is applied thereto. This measure does not ensure the correct dose of evacuated substance. In the case of ophthalmological substances, it is neither viable nor practical to have to visualise the amount of movement that is applied to a small outer rotating actuator, when at the same time the dose of substance is being dispensed directly onto an eye.

Patent document CN 209316515 teaches a dispenser that is conceptually similar to that which is disclosed in US 7309184 but the outer actuator of which is a push-button, loaded by an elastic element, which follows an oblique linear guide when actuated. This actuation results in a combination of forward translation and clockwise rotation movements, when the user presses the push-button, and backward translation and counterclockwise rotation movements, when the user allows the elastic element to return the push-button to its original, standby position.

This type of dispenser has the drawback that the push-button must be mechanically related to the piston with a complex system so that the push-button transmits push to the piston only when the push-button is moved forward by a user, but not when it is moved backwards, by the elastic element. Patent document CN204863997 proposes equipping the push-button with pins that interlock into a series of teeth inscribed in the piston and that relate by way of a ratchet so that only when the push-button moves forward do the pins and the teeth interlock mutually transmitting the rotation movement of the push-button to the piston, causing same to descend along a threaded guide. This is a solution that does not solve the aforementioned problems of manufacturing tolerances when the dispenser is small. This solution is also expensive and complicated, which also makes correct assembly between the components difficult.

Moreover, the devices described suffer from other drawbacks related to the type or amount of movement that is required to be applied in the same direction to the actuator that moves the component threadedly coupled with the piston to move the piston.

To facilitate the handling of the dispenser, it is of interest that the actuator should not rotate.

To facilitate the handling of the dispenser, it is also of interest that the movement stroke of the actuator is short.

An objective of the present invention is therefore a compact dispenser, easily manoeuvrable and capable of dispensing exact doses of a substance.

Further objectives of the present invention are a dispenser with easily assembled parts, the solution of which to secure a translational and non-return movement of a piston is applicable or compatible with dispensers that follow different dispensing principles, either of the airless pump of pressure pump type.

### Description of the invention

A dispenser is proposed according to claim 1.

The dispenser comprises a receptacle for storing the substance to be dispensed with a piston telescopically fitted into the receptacle to be moved, prevented from rotating by an anti-rotating mechanism, within said receptacle.

The piston is threadedly coupled to a rotating sleeve about an axis of rotation and the dispenser has an actuator, manually operable for its movement, and transmission means that connect the actuator with the rotating sleeve so that a movement of the actuator transmits a rotating movement of the rotating sleeve which in turn produces the movement of the piston within the receptacle.

In essence, the dispenser is characterised in that the actuator is an actuator that can be moved forward and backward in a first and a second direction of movement, respectively, between two stroke stops; and in that the transmission means comprise a pushing part in the actuator and a pushed part in the rotating sleeve, the pushing and pushed parts being configured so that the pushed part receives a push with a component that causes a rotation of the sleeve in the direction that moves the piston against the substance stored in the receptacle when the actuator is moved between its stroke stops, both forward and backward.

Throughout this specification, the term "comprises" or variations such as "has" or "with", will be understood to imply the inclusion of an element or group of elements mentioned, but not the exclusion of any other element or group of elements.

In accordance with the essence of the invention, instead of using solutions in which a double-acting actuator ensures the pulling during motion of a rotating sleeve that moves a piston during translation only when the actuator is moved in one direction, the inventors have decided to use a solution in which the double-acting actuator pulls the rotating sleeve during motion both when it is moved forward, in a first direction, and when it is moved backward, in a second direction opposite to the first.

Advantageously, it is not required to equip the dispenser with complex ratchet mechanisms or the like.

Also advantageously, a linear displacement of the piston towards the substance stored in the receptacle can be produced with more compact transmission means, as will be explained in more detail below.

A simple yet effective mechanical solution for the transmission means is the subject of a variant of the invention and is based on combining an axial cam, also sometimes called a drum cam, and at least one protruding member that mechanically interacts with the axial cam.

Advantageously, and as provided for by the invention, the axial cam can be provided in one of the pushing or pushed parts; and the at least one protruding member being provided on the other of the pushing or pushed parts.

In a variant of the invention, the rotating sleeve is cylindrical and has an outer wall in which the pushed part of the transmission means is formed or to which it is solidly attached; and the actuator is applied on the outside on the rotating sleeve and has an inner wall in which the pushing part of the transmission means is formed or to which it is solidly attached.

Preferably, although optional, the actuator is tubular and surrounds or sheaths the rotating sleeve on the outside.

Preferably, although optional, the actuator is a linear actuator, i.e., it is an actuator that can be moved in a straight direction, this being parallel to the axis of rotation of the rotating sleeve. Advantageously, this facilitates the manoeuvrability of the dispenser. Pressing an actuator is always easier than rotating an actuator.

In a variant of the invention, the axial cam can be formed or be solidly attached to the rotating sleeve, and be the pushed part, and the at least one protruding member can be formed or be solidly attached to the actuator, and be the pushing part. Alternatively, however, it is also conceived that the axial cam is formed or is solidly attached to the actuator, and is the pushing part, and that the at least one protruding member is formed or is solidly attached to the rotating sleeve, and is the pushed part.

In an embodiment of the invention according to the first option, wherein the axial cam is the pushed portion, the cam, which may be a raised formation on the outer face of the rotating sleeve, has a repeating profile with ascending ramps and descending ramps. In this embodiment of the invention the axial cam interacts with, specifically receives the push from, a first and a second type of protruding members, which are the pushing part, which are formed on the inner wall of the actuator. The assembly thus forms an inversion of axial cam and reciprocating translational follower, because the reciprocating follower is the active element here and the axial cam is the driven element. Specifically, the movement of the actuator in a direction parallel to the axis of rotation of the rotating sleeve, and thereby its protruding members, causes the rotation of said rotating sleeve.

The expression a first and a second type of protruding members should be interpreted as meaning that there are one or more protruding members of a first type and one or more protruding members of a second type.

In any case, it is another feature of the embodiment that has been described that the protruding member or members of the first type are axially and angularly offset in relation to the protruding member or members of the second type and that this offset is in accordance with the ascending and descending ramps of the axial cam, so that the protruding member or members of the first type interact only with ascending ramps of the axial cam when the forward actuator moves between its stroke stops and the protruding member or members of the second type interact only with descending ramps of the axial cam as the actuator moves backward between its stroke stops.

In a particular form of embodiment, the profile of the axial cam consists of an aligned succession of arrowheads all oriented towards the same direction, the opposite to the direction of rotation that is transmitted to the rotating sleeve, and joined by separate transitional segments, which connect the tip of an arrow with the base of the arrow that is adjacent thereto.

Optionally, but preferably, the axial cam has at least a number *n* of transversal passages which is equal to the number *n* of protruding members of the first type, the purpose of which is to allow, in an assembly manoeuvre of the rotating sleeve with the actuator, to be able to pass each protruding member of a first type through a corresponding passage from one side to the other of the axial cam, i.e., from one side to the other of the raised formation that forms said axial cam. Preferably, in that particular form of embodiment in which the profile of the axial cam consists of an aligned succession of arrowheads all oriented towards the same direction of rotation, the passages are located coincident with the transition sectors between the arrows.

In another variant embodiment of the invention in which the axial cam is the pushed part, the axial cam is formed by an annular guide that develops in a zig-zag pattern segments of a first upper guide wall defining ascending ramps and segments of a second lower guide wall defining descending ramps; and the at least one protruding member is formed on the inner wall of the actuator and is inserted into the guide.

As far as the anti-rotation mechanism is concerned, the invention contemplates various embodiments.

In one embodiment, the receptacle is formed and laterally closed by the juxtaposition to the head between a fixed shell casing and the rotating sleeve. The shell casing closes the receptacle on top, although it has an outlet that connects with valve means; and the rotating sleeve closes the receptacle at the bottom by means of the piston threadedly coupled therein.

In this embodiment, the anti-rotation mechanism comprises in the shell casing at least one, preferably several, fingers that extend longitudinally from a perimeter area of said shell casing and that form a sort of cage that encloses the piston; and in the piston, corresponding axial channels or grooves provided on an outer face thereof, each groove tightly receiving the body of one of said fingers. Said reception must be sufficient so that the fingers do not protrude from the threaded thread provided on the piston for its threaded coupling with the rotating sleeve.

The invention provides that the dispenser comprises a closure part and that this and the finger or fingers of the shell casing are configured for their mutual pressure coupling, firmly, in a state of assembly of the dispenser forming a package with the rotating sleeve, said rotating sleeve being tight but able to rotate between the closure part and the shell casing and outside the fingers of the shell casing.

The aforementioned mutual coupling can be achieved in various manners. In a variant of the invention, the coupling between the closure part and the distal end of the finger or fingers of the shell casing is produced by elastic restoring force, exerted by the finger or fingers of the shell casing against the closure part, retention projections of at least one finger of the shell casing crimping in a coupling recess of the closure part, or vice versa, i.e., at least one retention projection of the closure part crimping in a recess of a finger of the shell casing.

Preferably, the number of fingers is at least three and they are angularly equidistant.

In any case, according to a feature of interest, the rotating sleeve has on its inner wall and longitudinally a threaded coupling portion for coupling with the piston, provided for this purpose with a threaded thread; and an assembly portion, devoid of threaded thread, leaving space to accommodate, applied against the surface of said inner wall of the rotating sleeve, a thicker portion of the body of the fingers of the shell casing.

Among the possible construction variants, one is conceived that allows easy assembly of the components, without the help of auxiliary elements, such as screws or the like.

In a particular construction variant, applicable when the actuator is tubular and surrounds or sheaths the rotating sleeve, the actuator has a projection that cantilevers inwards that prevents its extraction by movement in a direction opposite to that of the receptacle. The cantilever projection is annular and can be continuous or discrete (intermittent) designed to abut the pushed part, in its case the axial cam, whatever the relative angular position between the actuator and the rotating sleeve.

The invention provides that the actuator is loaded by elastic means that tend to place it away from the shell casing. Then, the actuator must be pushed from the outside, directly or indirectly by a user, from a standby position in a forward movement, surpassing the force exerted on the same by these elastic means; and will automatically return to its standby position in a backward movement when the operator stops exerting the pushing force on the actuator.

In a variant, it is provided that the closure part assists in guiding the movement of the actuator, for example, when the movement of the actuator is linear and in the direction parallel to the axis of rotation of the rotating sleeve.

The dispenser is designed to be used in dispensers in which the evacuation of the stored substance is caused by the push of the piston.

The dispenser is intended for use in dispensers that practice other evacuation principles, including the airless pump principle.

In any case, it is of interest to preserve the properties of the stored substance and one factor that can contribute thereto is to guarantee that the stored substance remains stored under a pressure above atmospheric pressure, to prevent the entry of contaminants from the outside.

In this sense, the invention is compatible with means that seek to subject the substance stored in the receptacle to a pressure above atmospheric pressure.

In a variant of the invention, the piston has a head oriented towards the inside of the receptacle and on said head a pressurising body is mounted, axially movable with respect to the piston, subjected to the action of elastic means that tend to arrange the same towards the inside of the receptacle and against the substance stored therein.

In a specific embodiment, the pressurising body has a discoidal cover, sized according to the inner perimeter of the receptacle, from which one or more legs extend in the direction of the piston body; and the piston is provided with a number of guides, for example, in the form of axial holes, at least equal to the number of legs and sized to receive and guide the movement of the legs of the pressurising body.

The aforementioned guides, or axial holes, and the leg or legs of the pressurising body can be configured to limit the stroke of the pressurising body towards the inside of the receptacle by means of a mechanical stop.

For example, one or more legs of the pressurising body may have at its distal end a harpoon projection sized to crimp with a step formed in the guides, or in the axial holes, provided in the piston.

As regards the valve means connected to the outlet of the receptacle, these can follow a varied operating principle without affecting the nature of the invention.

The valve means may consist of a check valve.

Valve means may be more complex. In this sense, the valve means may comprise an entire ejector system, for example, such as that described in patent document EP 3738677.

The dispenser ejector system described in EP 373877 is of the type that incorporates a drive mechanism to operate the ejector system. This same drive mechanism can be used to transmit movement to the actuator of the dispenser that is the object of the present invention.

### Brief description of the drawings

Figures 1 and 2 are a cross-sectional and exploded view, respectively, of a dispenser according to a variant of the invention.
Figures 3a and 3b each show the play formed by the actuator, the rotating sleeve and the piston, with the actuator and the rotating sleeve in a correlative engagement position: Figure 3a a side view and Figure 3b a cross-sectional view and according to the cutting plane FF.
Figures 4a and 4b show moments of an operation sequence of the same dispenser, transparency techniques having been used with respect to the actuator, to show what would be hidden by the same.
Figures 5a and 5b also show the actuator, the rotating sleeve and the piston at times corresponding to those illustrated in Figures 4a and 4b, respectively, according to a diametral cutting plane.
Figure 5c is an enlarged detail of the area indicated by a circle in Figure 5b.
Figures 6a and 6b are respective lateral and cross-sectional views according to the cutting plane CC, respectively, of the piston of the same dispenser.
Figure 7 is a lateral view of an actuator and rotating sleeve assembly of a dispenser according to another variant of the invention.

### Detailed description of the invention

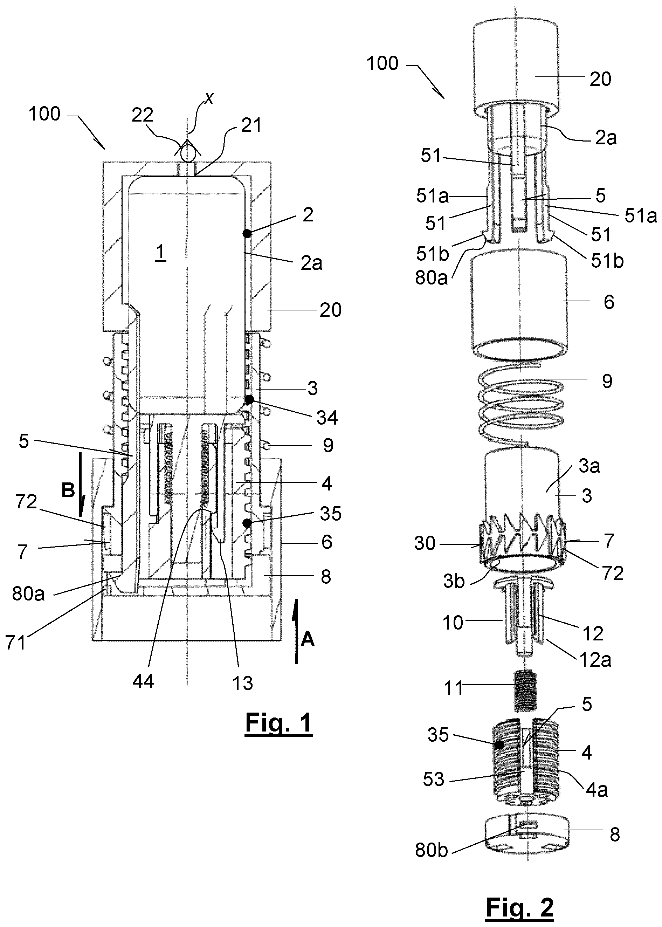

An exemplary embodiment of a dispenser 100 according to the invention is shown in Figures 1 and 2.

The dispenser 100 has a receptacle 2 for storing an ophthalmic substance 1 to be dispensed. This receptacle 2 is formed and laterally closed by the juxtaposition to the head between a shell casing 20 and a rotating sleeve 3. The shell casing 20 is generally cylindrical tubular in shape with a closed end that closes the receptacle 2 on top, although equipping the same with an outlet 21. The rotating sleeve 3 is also generally cylindrical tubular in shape, open at both ends, and houses a piston 4 that closes the receptacle 2 at the bottom. The facing mouths of the shell casing 20 and the rotating sleeve 3 are configured and sized to be applied against each other in the assembly position of the dispenser 100, which is that illustrated in Figure 1, defining a cylindrical receptacle 2 with a longitudinal axis coinciding with the axis of rotation x around which the rotating sleeve 3 rotates.

In the specific dispenser 100 the substance 1 fills a flexible container 2a. The flexible container 2a is collapsible and is inserted into the receptacle 2. The flexible container 2a is watertight, but in hydraulic connection with the outlet 21 of the receptacle 2 that connects with the outside via valve means 22, which are only schematically drawn as it is not an essential part of the invention.

The piston 4 is threadedly coupled to the rotating sleeve 3. On one hand, the rotating sleeve 3 has an inner wall 3b in which a threaded coupling portion for coupling with the piston 4 is distinguished, provided for this purpose with a threaded thread 34; on the other hand, the outer face 4a of the piston 4 is provided with a threaded thread 35, which matches the corresponding threaded thread 34 of the inner wall 3b of the rotating sleeve 3, which is interrupted by a series of axial grooves 53 which, as will be explained below, are part of an anti-rotation mechanism 5 the purpose of which is to provide only a translational movement of the piston 4 towards the substance 1 stored in the receptacle 2.

The dispenser 100 has an actuator 6. The actuator 6 is cylindrical tubular and surrounds or sheaths the rotating sleeve 3 on the outside. The actuator 6 can be moved forward and backward with respect to the shell casing 20 following a linear movement, in a direction parallel to the axis of rotation x of the rotating sleeve 3, in a first and a second direction, respectively, between two stroke stops.

The dispenser 100 has transmission means 7 that connect the actuator 6 with the rotating sleeve 3 so that the movement of the actuator 6 in both directions, the forward and backward direction between its two stroke stops, transmits a rotation movement to the rotating sleeve 3 which in turn causes, as it is prevented from rotating, the unscrewing of the piston 4 and its translational movement within the receptacle 2.

These transmission means 7 comprise a pushing part 71 in the actuator 6 and a push part 72 in the rotating sleeve 3.

The pushing 71 and pushed 72 parts are configured such that the pushed part 72 receives a push exerted by the pushing part 71 with a component normal to the axis of rotation x of the rotating sleeve 3 by applying a torque to said rotating sleeve 3 that makes it rotate around the axis of rotation x when the actuator 6 is moved both forward and backward, transmitting in both cases, the forward and backward, the rotation movement to the rotating sleeve 3 in the same direction of rotation D that corresponds to that which causes the unscrewing of the piston 4 and its translational movement towards the substance stored in the receptacle 2.

In the dispenser 100 of the exemplary embodiment, the rotating sleeve 3 has an outer wall 3a in which the pushed part 72 of the transmission means 7 is formed or solidly attached thereto; and the actuator 6 has an inner wall 6b in which the pushing part 71 of the transmission means 7 is formed or solidly attached thereto.

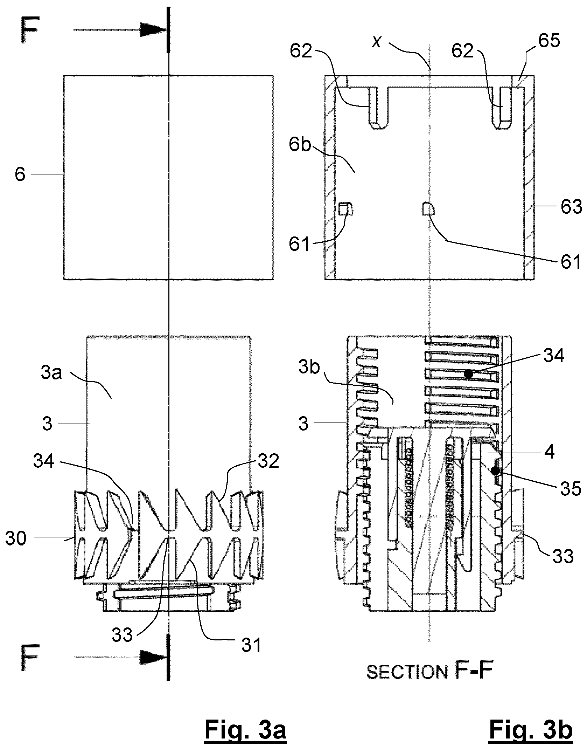

More specifically, as shown in Figures 3a and 3b, in the dispenser of the exemplary embodiment, the pushed part 72 of the transmission means 7 consist of an axial cam 30, which annularly extends all the way around the rotating sleeve 3; and the pusher part 71 comprises protruding members 61, 62 of a first and a second type that mechanically interact with the axial cam 30.

Even more specifically, the axial cam 30 is formed by a raised formation the profile of which consists of an aligned succession of equal arrowheads, all oriented in the same direction and joined by separate transitional sectors 33, which connect the tip of the arrow with the base of the arrow that is adjacent thereto, all giving rise to a repetitive profile with ascending ramps 31 and descending ramps 32.

In an associated manner, the first and second types of protruding members 61, 62 are axially and angularly offset in the actuator 6 in relation to the ascending 31 and descending 32 ramps of the axial cam 30, so that the protruding members 61 of the first type are intended to interact only with ascending ramps 31 of the axial cam 30 when the forward actuator 6 moves between its stroke stops and the protruding members 62 of the second type are intended to interact only with descending ramps 32 of the axial cam 30 when the actuator 6 moves backward between its stroke stops.

The expression ascending ramps and descending ramps should be interpreted as presenting positive and negative inclinations, respectively, with respect to the direction along which the axial cam extends. Ramps can be straight, curved or combine straight and curved segments.

The dispenser 100 is specially prepared so that its parts can be easily assembled.

As far as the anti-rotation mechanism 5 is concerned, in the example the shell casing 20 is provided with three fingers 51 that extend longitudinally, i.e., in the direction of the axis of rotation x, from a perimeter area thereof, angularly equidistant, and forming a sort of cage that encloses the piston 4. These fingers 51 fit into the corresponding axial grooves 53 provided on the outer face 4a of the piston 4, each groove 53 tightly receiving the body 51a of one of said fingers 51 without protruding or bulging from the threaded thread 35.

The assembly of the dispenser 100 is achieved by providing a closure part 8 couplable to the fingers 51 and packing the rotating sleeve 3 against the shell casing 20, although able to rotate with respect thereto.

In the example, the closure part 8 and the distal end 51b of the fingers 51 are configured for their mutual snap coupling in an assembly state of the dispenser 100.

More specifically, the mutual coupling between the closure part 8 and the distal end 51b of the fingers 51 of the shell casing 20 is produced by elastic restoring force, exerted by the fingers 51 of the shell casing 20 against the closure part 8, retention projections 80a of the fingers 51 of the shell casing 20 crimping in coupling recesses 80b of the closure part 8.

It should be noted that in the rotating sleeve 3 there is a longitudinal portion of threaded coupling with the piston 4, provided for this purpose with threaded thread 34; and also a longitudinal assembly portion, devoid of threaded thread, the purpose of which is to leave space to accommodate, applied against the surface of said inner wall 3b of the rotating sleeve 3, a thicker portion of the body 51a of the fingers 51 of the shell casing 20, the length of which must be sufficient to reach the closure part 8 at its distal ends.

In the package formed, the actuator 6 is trapped, with longitudinal play, between the shell casing 20 and the rotating sleeve 3, guided in its longitudinal movement by the outer wall 3a of the rotating sleeve 3.

The invention provides that the closure part 8 contributes to the guidance of the actuator 6, to follow a translational movement only.

In the example, the actuator 6 is loaded by elastic means 9 in the direction of the closure part 8. More specifically, the elastic means 9 are made up of a spring that acts by compression and rests against the shell casing 20 and the actuator 6 itself.

In the example, the actuator 6 has a projection 65 that cantilevers inwards that allows the placement of the actuator 6 on the rotating sleeve 3 from its upper portion but that prevents the extraction of the actuator 6 by sliding the same towards the closure part 8,by abutment of the projection 65 against the pushed part 72, in this case by stop against the axial cam 30 of the rotating sleeve 3.

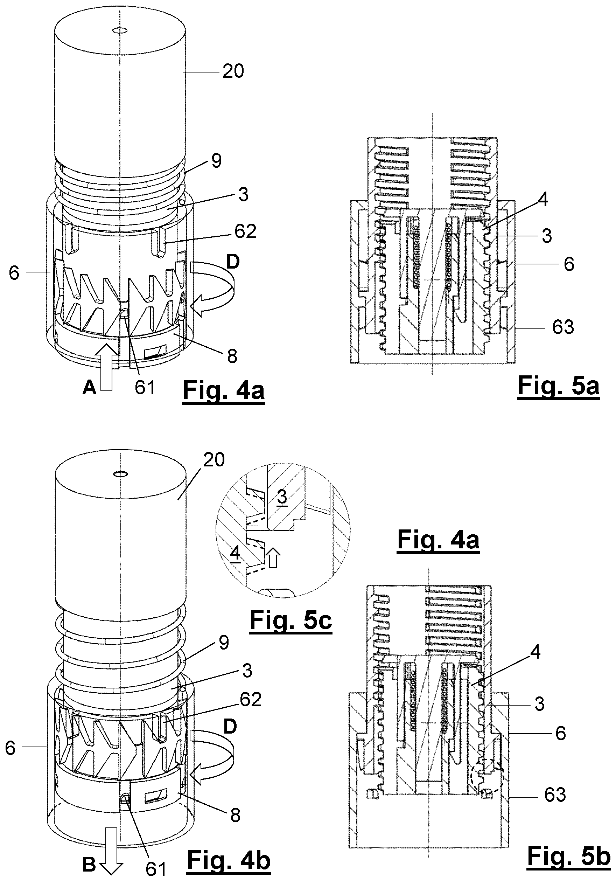

However, the operation of dispenser 100 is as follows:
- Starting from the standby situation, the one shown in Fig. 1, with the actuator 6 applied against its lower stroke stop by the effect of the spring 9, the actuator 6 is moved forward, in a first direction A, ascending with reference to the drawings, against its upper stroke stop, overcoming the force exerted by the spring 9 on same.
   Note that, in a first movement phase, the protruding members 62 of the second type that remained engaged in the axial cam 30 are dislodged; while in a second movement phase, the protruding means 62 of the second type being disengaged from the axial cam 30 - here lies the need for axial offset between the protruding members 61 and 62 of the first and the second type, respectively - the protruding members 61 of the first type exert a push on the ascending ramps 31 of a series of arrowheads of the axial cam 30 producing a rotation of the rotating sleeve 3, in clockwise direction D with reference to the drawings, which in turn produces a translational movement of piston 4, ascending with reference to the drawings. Said second movement phase will end when the protruding members 61 of the first type reach the position illustrated in Fig. 4a, which is the upper stroke stop of the actuator 6 by contact of the protruding means 61 of the first type against transitional sectors 33 of the axial cam 30.
- From the position illustrated in Figure 4a, an outer force no longer being exerted on the actuator 6, it automatically moves backwards, in a second direction B, downward with reference to the drawings, due to the effect of the spring 9 against its lower stroke stop.
   Note that, in a first movement phase, the protruding members 61 of the second type that remained engaged in the axial cam 30 are now dislodged; while in a second movement phase, once the protruding means 61 of the second type of the axial cam 30 are disengaged, the protruding members 62 of the second type exert a push on the descending ramps 32 of arrowheads of the axial cam 30 - here lies the need for angular offset between the protruding members 61 and 62 of the first and second type, respectively - causing a rotation of the rotating sleeve 3, maintaining the clockwise direction D with reference to the drawings, which in turn causes a new translational movement of the piston 4, ascending again in reference to the drawings. Said second movement phase will end when the protruding members 61 of the first type reach the position illustrated in Figure 4b, which is the lower stroke stop of the actuator 6 by contact of the protruding means 62 of the second type against transitional sectors 33 of the axial cam 30.

The exemplary embodiment of the dispenser of the invention stores an ophthalmic substance. Consequently, the ascending stroke of the piston 4 that defines the reduction in volume of the receptacle 2 that is related to the dose of substance evacuated, is small.

In the exemplary embodiment of the dispenser of the invention, the stroke of the actuator 6 is 8.4 mm; and its movement between stroke stops causes a displacement of the piston 4 of 0.07 mm. Consequently, the forward and backward movement of the actuator 6 causes a total movement of the piston 4 of 0.14 mm. This represents an approximate dose of evacuated substance of 0.04 ml.

Figure 5c aims to illustrate this small movement of the piston 4, specifically the movement that occurs when the actuator 6 moves between its stroke stops and more specifically between the positions represented in Figures 4a and 4b. Figure 5c shows the thread profile of the piston 4 in dashed lines in the position it adopted at the beginning of its movement. This is an explanatory representation and therefore the illustrated movement of the piston 4 in proportion to other components of the dispenser may not be realistic.

In the exemplary embodiment of the dispenser of the invention, the total rotation transmitted to the rotating sleeve 3 with the forward and backward movement of the actuator 6 is 22.5°.

Other aspects of interest of the dispenser 100 follow below.

Producing a rotation of the rotating sleeve 3 both in the forward movement and in the backward movement of the actuator 6, using two ramps of the axial cam with inverted slope with respect to each other, causing in both cases the translation of the piston 4 towards the stored substance displays several simultaneous effects that have to do with compactness and smooth operation.

And achieving a certain translational movement of the piston 4 using a single ramp on the axial cam may require that said ramp must be either very long (harming compactness) or with very little slope (harming smoothness of operation - since technically it translates into a push with a smaller component in the direction normal to the axis of rotation x of the rotating sleeve).

Now, highlighting again other aspects of the dispenser 100, it should be noted that the actuator 6 is sized to conceal the rotating sleeve 3. Specifically, the skirt 63 of the actuator 6 has a height that conceals the rotating sleeve 3 even when the actuator 6 is moved against its upper stroke stop. This makes it difficult for a user to access the rotating sleeve 3 and manipulate it directly. Consequently, this is a measure that improves the safety of use of the dispenser.

The axial cam 30 has a number *n* of transversal passages 34 equal to the number *n* of protruding members 61 of the first type, the purpose of which is to allow, in an assembly manoeuvre of the rotating sleeve 30 with the actuator 6, to be able to pass each protruding member 61 of a first type through a corresponding passage 34 from one side of the axial cam 3 to the other, in the example from the side of the shell casing 20 towards the side where the closure part 8 will be coupled. In an assembly manoeuvre of the dispenser 100, the actuator 6 can be placed outside the rotating sleeve 3 and each protruding member 61 of a first type is made to pass through a corresponding passage 34 from one side to the other of the axial cam 30; and then placing the spring 9 before closing the receptacle by placing the shell casing 20 and forming the package with the closure part 8. The same spring 9 can be used as a means of upper safety stop of a movement of the actuator 6 when normally operating the dispenser 100 to evacuate a dose of the substance, all the protruding members 61 of a first type coincide angularly with passage 34 of the axial cam 30 when the forward actuator moves, preventing said protruding members 61 from passing to the other side of the axial cam 30.

The translational movement of the piston 4 towards the substance stored in the receptacle 2 can cause an overpressure sufficient to open the normally closed valve means 22 connected to the outlet 21 of the receptacle. The valve means 22 may then be formed by a common check valve.

The forward movement of the actuator 6 can be carried out manually by the user, directly exerting pressure on the actuator 6.

The forward movement of the actuator 6 can be carried out manually by the user, although operating on a drive mechanism the movement of which is transmitted in any way to the actuator 6. A drive mechanism may be, by way of example, such as that which is described in patent document EP 3738677 which is used to drive an ejector system for a substance stored in the dispenser.

The backward movement of the actuator 6 may be manual, but it is preferably automatic and, in the exemplified case, the actuator 6 is loaded by elastic means for this purpose, all of this as has been exemplified in the dispenser 100.

In any case, the forward and backward movement between its stroke stops of the actuator 6 corresponds to a decrease in the volume of the receptacle 2, equal to or proportional to the volume of the substance evacuated from the receptacle 2.

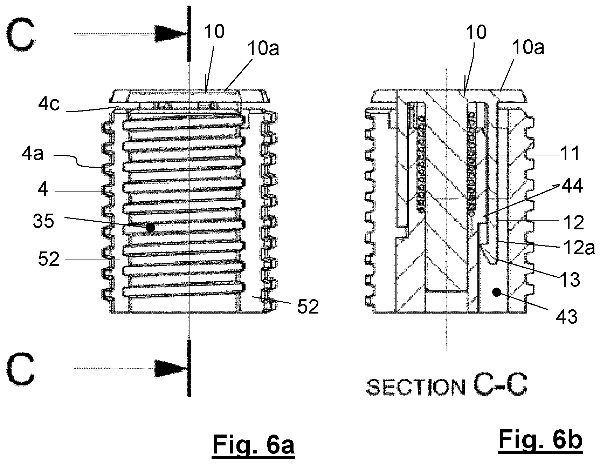

Additionally, the dispenser 100 is equipped with means that seek to subject the substance 1 stored in the receptacle 2 to a pressure above atmospheric pressure, as illustrated in Figures 6a and 6b.

Specifically, the piston 4 has a head 4c, oriented towards the inside of the receptacle 2 on which a pressurising body 10 is mounted axially movable with respect to the piston 4, subjected to the action of elastic means 11 that tend to arrange the same towards the inside of the receptacle 2 and against the substance stored therein. In the example, the elastic means 11 are formed by a spring that acts under compression, supported against the piston 4 and the pressurising body 10.

In the exemplary dispenser 100, the pressurising body 10 has a discoidal cover 10a, sized according to the inner perimeter of the receptacle 2, from which four legs 12 extend in the direction of the body of the piston 4, and the piston 4 is provided with a series of guides 43 here in the form of axial holes sized to receive and guide the movement of the legs 12 of the pressurising body 10.

The axial holes that act as guides 43 and the legs 12 of the pressurising body 10 are configured to limit the stroke of the pressurising body 10 towards the inside of the receptacle 2 by means of a mechanical stop.

The mechanical stop can be implemented in several ways.

In the exemplary dispenser 100, the legs 12 of the pressurising body 10 have at their distal end 12a a projection 13 sized to abut a step 44 formed in a wall of the guides 43.

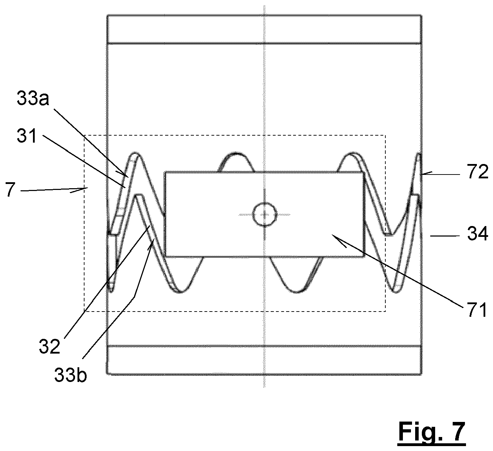

Figure 7 schematically shows an alternative for the transmission means 7, compatible with the dispenser 100 described above.

This variant of the transmission means 7 shares with that of the dispenser 100 that the pushed part 72 is formed or is solidly attached to the rotating sleeve 3 and comprises an axial cam 30; and that the pusher part 71 is formed or is solidly attached to the actuator 6.

In order to facilitate understanding, the actuator 6 is only partially represented in Figure 7. The actuator 6 may have a similar configuration to that described for the dispenser 100, in the sense that it may be a cylindrical tubular part that surrounds or covers the rotating sleeve 3.

It is particular to the transmission means 7 of Figure 7 that the axial cam 30 is formed by an annular guide 34 that develops in a zig-zag pattern segments of a first upper guide wall 33a defining the ascending ramps 31 and segments of a second lower guide wall 33b defining the descending ramps 32, and that in this case the pushing part 71 is at least one protruding member 31 that is inserted into the guide 33.

## Claims

1. A dispenser (100) for doses of liquid or pasty substances (1) comprising
- a receptacle (2) for storing the substance to be dispensed, with an outlet (21) connected to valve means (22),
- a piston (4) telescopically fitted into the receptacle (2) to move, prevented from rotating by an anti-rotation mechanism (5), inside the receptacle,
- a rotating sleeve (3) around an axis of rotation (x), threadedly coupled to the piston (4),
- an actuator (6), manually operable for its movement,
- transmission means (7) that connect the actuator (6) with the rotating sleeve (3) so that a movement of the actuator (6) transmits a rotation movement to the rotating sleeve (3) which in turn causes the movement of the piston (4) inside the receptacle (2),
**characterised in that**
the actuator (6) is an actuator able to move forward and backward in a first (A) and a second (B) direction of movement, respectively, between two stroke stops; and **in that**
the transmission means (7) comprise a pushing part (71) in the actuator (6) and a pushed part (72) in the rotating sleeve (3), wherein the pushing (71) and pushed parts (71, 72) are configured so that the pushed part (72) receives a push with a component that causes a rotation of the rotating sleeve (3) in the direction that moves the piston (4) against the substance stored in the receptacle (2) when the actuator (6) is moved between both its forward and return stroke stops.

2. The dispenser (100) according to claim 1, **characterised in that** one of the pushing part (71) or the pushed part (72) of the transmission means (7) is an axial cam (30), which annularly extends a full turn around the axis of rotation (x) of the rotating sleeve (3); and the other of the pushing part (71) or the pushed part (72) of the transmission means (7) comprises at least one protruding member (61) that interacts mechanically with the axial cam (30).

3. The dispenser (100) according to claim 2, **characterised in that** the pushing part (71) of the transmission means (7) has the axial cam (30), which is formed or is solidly attached to the actuator (6); and **in that** the pushed part (72) has at least one protruding member (61), which is formed or solidly attached to the rotating sleeve (3).

4. The dispenser (100) according to claims 2 or 3, **characterised in that**
- the axial cam (30) has a repetitive profile with ascending (31) and descending ramps (32), and **in that** the at least one protruding member (61) comprises
- a first and a second type of protruding members (61, 62) that are axially and angularly offset in relation to the ascending (31) and descending (32) ramps of the axial cam (30), so that
- the protruding member or members (61) of the first type interact only with ascending ramps (31) of the axial cam when the actuator (6) moves forward between its stroke stops and the protruding member or members (62) of the second type interact only with descending ramps (32) of the axial cam when the actuator (6) moves backward between its stroke stops.

5. The dispenser (100) according to claim 4, **characterised in that** the axial cam (30) is formed by a raised formation the profile of which consists of an aligned succession of arrowheads all oriented towards the same direction, the opposite to that of the direction of rotation of the rotating sleeve (3), and joined by respective transitional sectors (33), which connect the tip of an arrow with the base of the arrow that is adjacent thereto.

6. The dispenser (100) according to claim 5, **characterised in that** the axial cam (30) has at least one number *n* of transversal passages (34) that is equal to the number *n* of protruding members (61) of the first type, the purpose of which is to allow, in an assembly manoeuvre for assembling the rotating sleeve (3) with the actuator (6), to be able to pass each protruding member (61) of a first type through a corresponding passage (34) from one side to the other of the axial cam (30), that is, from one side to the other of the raised formation that forms said axial cam, or to the axial cam (30) from one side to the other of protruding members (31) of the first type.

7. The dispenser (100) according to claims 2 or 3, **characterised in that**
- the axial cam (30) is formed by an annular guide (34) that develops in a zig-zag pattern segments of a first upper guide wall (33a) defining ascending ramps (31) and segments of a second lower guide wall (33b) defining descending ramps (32), and **in that**
- the at least one protruding member (61) is inserted into the guide (34).

8. The dispenser (100) according to any one of claims 2 to 7, **characterised in that** the actuator (6) is movable between its stroke stops following a straight path, parallel to the axis of rotation (x) of the rotating sleeve (3); and **in that** it is subjected to the action of elastic means (9) that tend to place the same against one of its stroke stops.

9. The dispenser (100) according to any one of claims 2 to 8, **characterised in that** the receptacle (2) is closed laterally by the juxtaposition to the head between a shell casing (20), which closes the receptacle at the top with the outlet (21), and the rotating sleeve (3), which closes the receptacle at the bottom with the threadedly coupled piston (4) inside.

10. The dispenser (100) according to claim 9, **characterised in that** the anti-rotation mechanism (5) comprises in the shell casing (20) several fingers (51) that extend longitudinally from a perimeter area of said shell casing (20) and that form a sort of cage that encloses the piston (4); and in the piston (4), corresponding axial grooves (53) provided on an outer face (4a) thereof, each groove (53) tightly receiving the body (51a) of one of the aforementioned fingers (51).

11. The dispenser (100) according to claim 10, **characterised in that** it comprises a closure part (8), **in that** the closure part (8) and the distal end (51b) of the fingers (51) are configured for their mutual pressure coupling in a state of assembly of the dispenser (100) forming a package with the rotating sleeve (3), said rotating sleeve (3) being tight between the closure part (8) and the shell casing (20), although able to rotate around its axis of rotation (x), and outside the fingers (51) of the shell casing (20).

12. The dispenser (100) according to any one of the preceding claims, **characterised in that** the rotating sleeve (3) is tubular, has an inner wall (3b) in which a longitudinal portion of threaded coupling with the piston (4) is distinguished, provided for this purpose with a threaded thread (34); and a longitudinal assembly portion, devoid of threaded thread.

13. The dispenser (100) according to any one of the preceding claims, **characterised in that** the piston (4) has a head (4c), facing towards the inside of the receptacle (2); and **in that** on the head (4c) a pressurising body (10) is mounted axially movable with respect to the piston (4), subjected to the action of elastic means (11) that tend to arrange the same towards the inside of the receptacle (2) and against the substance stored therein.

14. The dispenser (100) according to claim 13, **characterised in that** the pressurising body (10) has a discoidal cover (10a), sized according to the inner perimeter of the receptacle (2), from which legs (12) extend in the direction of the piston (4) body; and **in that** the piston (4) is provided with a series of guides (43) sized for receiving the plug and guide the movement of the legs (12) of the pressurising body (10).

15. The dispenser (100) according to claim 14, **characterised in that** the guides (43) and the legs (12) of the pressurising body (10) are configured to limit by mechanical stop the stroke of the pressurising body (10) towards the inside of the receptacle (2) and against the substance stored therein.
